# EUROPEAN PATENT APPLICATION

(11) **EP 2 977 048 A1**
(43) Date of publication of application: **27.01.2016**
(21) Application number: 14764870.3
(22) Date of filing: 11.03.2014
(51) Int. Cl.: A61K 31/41, A61K 31/505, A61P 3/06, A61P 3/00

(54) **COMPLEX PREPARATION INCLUDING VALSARTAN AND ROSUVASTATIN CALCIUM AND MANUFACTURING METHOD THEREFOR**

(30) Priority: 12.03.2013 KR 20130026146
(71) Applicant: LG Life Sciences Ltd., Seoul 110-783 (KR)
(72) Inventor: SONG, Jeong Uk, Daejeon 305-738 (KR); KIM, Geun Tae, Daejeon 305-738 (KR); KIM, Sun Il, Daejeon 305-738 (KR); LEE, Ki Kon, Daejeon 305-738 (KR)
(74) Representative: Gillard, Richard Edward
(86) International application number: PCT/KR2014/002008
(87) International publication number: WO 2014/142521

(57) **Abstract**

The present invention relates to a pharmaceutical composition including valsartan and rosuvastatin calcium as active ingredients, and a manufacturing method therefor. The pharmaceutical composition including valsartan and rosuvastatin calcium according to the present invention can alleviate side effects of the existing single preparations and simultaneously improve compliance of patients having a cardiovascular disease, a hyperlipidemic disease and a complex disease thereof, and increases treatment and prevention effects, and in particular, obtains synergistic effects in treating hyperlipidemia, and enhances the dissolution rate of valsartan and rosuvastatin calcium and treatment effects by granulating valsartan and mixing rosuvastatin calcium.

## Description

### [TECHNICAL FIELD]

The present invention relates to a pharmaceutical composition comprising valsartan and rosuvastatin calcium as active ingredients, and a method for preparing the same.

### [BACKGROUND ART]

Hypertension and hyperlipidemia are well known as important factors of atherosclerosis. According to Interheart research, hyperlipidemia's attributable risk of atherosclerosis is 49% which is the highest, and hypertension's attributable risk is 18% which is also an important risk factor. Among adult diseases, hypertension-which has the highest attributable risk of atherosclerosis after hyperlipidemia-is the disease with the largest number of sufferers.

Hypertension and hyperlipidemia concur in many cases, and accordingly the rate of prescribing a hypertension-treating agent and a hyperlipidemia-treating agent in combination is high. Because most patients having hypertension and hyperlipidemia should take several drugs in large amounts for a long time, drug fidelity is very important. If drug fidelity is low, recurrence of myocardial infarction increases. In particular, many patients show low fidelity to a lipid-lowering agent. According to a research result, a patient who had taken one pill showed fidelity of about 20% higher than that of a patient who had taken two pills. Accordingly, if a complex formulation for hypertension and hyperlipidemia having complex ingredients is developed, it can provide the excellent advantage of reducing the patients' burden of the amount and cost of the drug(s) to be taken.

In consideration of the fact that hypertension and hyperlipidemia are important factors of atherosclerosis, the present inventors have conducted intensive researches to develop a new composition, through which a hypertension-treating agent and a hyperlipidemia-treating agent are administered in combination and act synergistically to improve the treatment effect, mitigate a side effect(s), and improve compliance of patients having both hypertension and hyperlipidemia. As a result thereof, the present inventors have found that combined use of the known valsartan and rosuvastatin calcium can achieve the purposes of providing a synergistic effect in hypertension treatment, improving the treatment effect on a cardiovascular disease and hyperlipidemia, mitigating a side effect(s) and improving compliance of patients. In particular, the present inventors have found that a complex formulation of a specific structure containing these drugs can be preferably used for such purposes, and have arrived at the completion of the present invention.

### [DETAILED DESCRIPTION OF THE INVENTION]

### [TECHNICAL PURPOSES]

One purpose of the present invention is to provide a pharmaceutical composition for preventing or treating a cardiovascular disease and a hyperlipidemic disease, which can improve the treatment effect, mitigate a side effect(s) and improve compliance of patients having both hypertension and hyperlipidemia by combined use of a hypertension-treating agent and a hyperlipidemia-treating agent having different action mechanisms from each other.

The other purpose of the present invention is to provide a method for preparing a complex formulation for preventing or treating a cardiovascular disease and a hyperlipidemic disease, which comprises a hypertension-treating agent and a hyperlipidemia-treating agent having different action mechanisms from each other.

Another purpose of the present invention is to provide a pharmaceutical composition for treating hypertension, which exhibits a synergistic effect by combined use of a hypertension-treating agent and a hyperlipidemia-treating agent having different action mechanisms from each other.

### [TECHNICAL MEANS]

To achieve said purpose, the present invention provides a pharmaceutical composition for preventing or treating a cardiovascular disease and a hyperlipidemic disease, comprising valsartan and rosuvastatin calcium as active ingredients.

In the pharmaceutical composition of the present invention, the valsartan and rosuvastatin calcium may be present in a state of being separated, and in particular, preferably the rosuvastatin calcium is in a state of being mixed to valsartan granule, and more preferably the pharmaceutical composition is in a form of bilayer tablet where each of the valsartan and rosuvastatin calcium is present in each of the separate layers.

Furthermore, the pharmaceutical composition of the present invention may comprise the rosuvastatin and valsartan in a weight ratio of from 1:2 to 1:32, and a weight ratio of 1:4, 1:8, 1:16 or 1:32 is particularly preferred.

To achieve said other purpose, the present invention provides a method for preparing a pharmaceutical composition for preventing or treating a cardiovascular disease and a hyperlipidemic disease, comprising the steps of: (a) granulating a mixture of valsartan and a pharmaceutically acceptable additive; and (b) mixing rosuvastatin calcium and a pharmaceutically acceptable additive to the valsartan granule of step (a).

In the preparation method according to the present invention, preferably a mixture of 1 part by weight of the valsartan and 1 to 3 parts by weight of the pharmaceutically acceptable additive is granulated in said step (a), and preferably 1 part by weight of the rosuvastatin calcium and 1 to 10 parts by weight of the pharmaceutically acceptable additive are mixed in said step (b).

To achieve said another purpose, the present invention provides a pharmaceutical composition for synergistically treating hypertension, comprising valsartan and rosuvastatin calcium as active ingredients. Preferably, the composition is a tablet and comprises, per tablet, 160 mg of valsartan and 20 mg of rosuvastatin.

Furthermore, the present invention includes use of a pharmaceutical composition comprising valsartan and rosuvastatin calcium as active ingredients for preventing or treating a cardiovascular disease and a hyperlipidemic disease.

In addition, the present invention provides a method of preventing or treating a cardiovascular disease and a hyperlipidemic disease, comprising a step of administering a pharmaceutical composition comprising valsartan and rosuvastatin calcium as active ingredients to a subject in need.

The present invention is explained in more detail below.

Valsartan, which is an angiotensin II receptor antagonist having a vasoconstriction and sodium retention effect, reduces blood pressure by vasodilation. Valsartan can be obtained by purchase of the commercially available Tareg^{®} tablet, or preparation according to a known method. For example, the whole contents of the method for preparing valsartan is disclosed in US 5,399,578. As well as its isolated form, valsartan can be used in any suitable salt form for the purpose of the present invention. In the U.S., as a hyperlipidemia-treating agent, valsartan is used in an initial dose of 80 to 160 mg, qd and can be prescribed in the maximum dose of 320 mg, qd. In Korea, valsartan is prescribed for patients with cardiac insufficiency or postmyocardial infarction syndrome in a dose of 160 mg, bid. However, if the blood pressure of the patient does not reach the desired value with the dose of 160 mg, qd prescription, valsartan is replaced with another drug(s). Valsartan is typically used in a dose of from about 40 mg to about 320 mg, preferably from about 80 mg to about 320 mg, and most preferably about 80 mg or about 160 mg, in a tablet.

Rosuvastatin calcium is an HMG CoA reductase inhibitor and is used in the treatment of primary hypercholesterolemia (type IIa including heterozygous familial hypercholesterolemia), complex hyperlipidemia (type IIb), primary dysbetalipoproteinemia (type III) and homozygous familial hypercholesterolemia. Prior to and during administration of this drug, patients should have a standard low-cholesterol diet and it must be maintained during the period of treatment. This drug may be taken at any time in a day regardless of meal consumption. The initial dose is 5 mg once a day, and if further reduction of LDL-cholesterol level is required, it can be adjusted to the maintenance dose and administered. The maintenance dose is 10 mg once a day, and almost all patients are controlled in this dose. The maintenance dose should be adjusted properly according to LDL-cholesterol level, treatment target and patient's response with an interval of 4 weeks or longer, and can be increased to the maximum of 20 mg a day. The risk of cardiovascular disease is reduced by the administration of this drug. Although there is no clinical evidence on coronary heart disease, for patients, who are men aged 50 years or older or women aged 60 years or older, and have 2 mg/L or higher of high sensitive C-reactive protein (hsCRP) and at least one or more cardiovascular disease risk factors (e.g., hypertension, low HDL-cholesterol level, smoking or family history of early-onset of coronary artery heart disease, etc.), the risk of stroke, the risk of myocardial infarction and the risk of arterial vessel angioplasty are reduced.

Rosuvastatin calcium is marketed under the commercial name of Crestor^{®} tab as formulated in a tablet containing 5 mg, 10 mg or 20 mg. Rosuvastatin calcium is commercially available from AstraZeneca (GB), and can be prepared according to the disclosure of US RE37314 and Korean Patent No. 10-0105432, of which the whole context is quoted herein. In addition, US 6,316,460 discloses a method for treating patients suffering from hyperlipidemia, comprising administering a combined formulation containing rosuvastatin calcium as the active ingredient.

In the present invention, by the combined use of valsartan and rosuvastatin calcium and through the synergistic interaction of the drugs, it is intended to strengthen the effect of preventing or treating a cardiovascular disease as compared with the existing single formulation, reduce the side effects of each of the drugs, and improve patient compliance. Accordingly, the pharmaceutical composition of the present invention comprising valsartan and rosuvastatin calcium can be used effectively for preventing or treating a cardiovascular disease and hyperlipidemia. Examples of the cardiovascular disease include angina pectoris, hypertension, arteriospasm, cardiac arrhythmia, cardiac hypertrophy, cerebral infarction, congestive heart failure, myocardial infarction, etc., but are not limited thereto.

However, if the complex formulation of the present invention comprising valsartan and rosuvastatin calcium is prepared by simply mixing the two drugs, there may be some problems caused by the difference between the inherent physical properties of valsartan and rosuvastatin calcium.

The first problem is gelling of valsartan. Valsartan begins to gel at pH 4.0 or lower due to its low solubility and dissolves very slowly. Such delayed dissolution causes delayed absorption of valsartan in the small intestine. In addition, gelling of valsartan can delay the dissolution of rosuvastatin calcium. This trouble can adversely affect the absorption of rosuvastatin calcium which is rapidly disintegrated and absorbed in the gastro-intestinal tract. Accordingly, it is necessary to develop a formulation without gelling of valsartan even under a low pH condition.

The second problem is that in an experiment of preparing the complex formulation by mixing valsartan and rosuvastatin calcium together and granulating the mixture, the phenomenon of lowering the dissolution of the drugs is observed and such a dissolution-lowering phenomenon seriously affects the absorption and bioavailability of the two drugs. That is, the efficacy of the complex formulation is expected to decrease due to the change in bioavailability, and thus a novel formulation process which can improve it is required.

In the present invention, when formulating valsartan and rosuvastatin calcium, a manner of granulation of valsartan and post-mixing of rosuvastatin calcium has been designed in order to overcome various problems according to the complex formulation process of valsartan and rosuvastatin calcium. The complex formulation according to this exhibits good dissolution rates for each of valsartan and rosuvastatin calcium.

As an embodiment according to the present invention, the complex formulation of valsartan and rosuvastatin calcium can be prepared by a method comprising the steps of: (a) granulating a mixture of valsartan and a pharmaceutically acceptable additive; and (b) mixing rosuvastatin calcium and a pharmaceutically acceptable additive to the valsartan granule of step (a).

For example, the complex formulation of the present invention can be prepared by mixing rosuvastatin calcium to the valsartan granule obtained in step (a) and pressing the mixture into a tablet. The complex formulation prepared as such exhibits higher dissolution rates of valsartan and rosuvastatin calcium, as compared with a tablet prepared by simply mixing valsartan and rosuvastatin calcium and wet-granulating the mixture.

As another concrete embodiment, the present invention provides a complex bilayer tablet of valsartan and rosuvastatin calcium. The complex bilayer tablet can be obtained by pressing the valsartan granules into a first tablet layer and then pressing the mixture of rosuvastatin calcium and an additive into a second tablet layer by using a bilayer tablet press.

In the complex formulation according to the present invention, the active ingredients, valsartan and rosuvastatin calcium, can be contained in a weight ratio of from 2 to 32 parts by weight of valsartan, based on 1 part by weight of rosuvastatin in the composition.

The pharmaceutical composition for preventing or treating a cardiovascular disease and hyperlipidemia according to the present invention may comprise a pharmaceutically acceptable carrier or additive in each of the valsartan granule and rosuvastatin calcium. Examples of the pharmaceutically acceptable carrier or additive include microcrystalline cellulose, lactose, low-substituted hydroxypropyl cellulose, disintegrating agents (e.g., croscarmellose sodium, crospovidone, sodium starch glycolate), and granulation binders (e.g., polyvinylpyrrolidone, hydroxypropyl cellulose) and calcium hydrogen phosphate. In addition, a lubricant such as colloidal silicon dioxide, hydrous silicon dioxide, magnesium stearate, sodium stearyl fumarate (commercial name: Pruv), glyceryl behenate (commercial name: Compritol 888), calcium stearate, stearic acid and talc may be included.

In the preparation method according to the present invention, when post-mixing rosuvastatin calcium, a pharmaceutically acceptable additive may be comprised in an amount of from 1 to 10 parts by weight based on 1 part by weight of rosuvastatin calcium, and the valsartan granule may comprise a pharmaceutically acceptable excipient in an amount of from 1 to 3 parts by weight based on 1 part by weight of valsartan. Such quantitative ratios are applied to all the formulations of the present invention including the separated granules. If the formulation has a ratio out of the above ranges, the tablets may become too small or too large, and the dissolution may be delayed or accelerated.

In addition, the complex formulation according to the present invention can be film-coated, and examples of coating agents available in the film coating layer include conventional ones, such as hydroxypropylmethyl cellulose, Opadry series, Eudragit series, etc. but are not limited thereto.

### [EFFECT OF THE INVENTION]

By the combination of the drugs having different action mechanisms from each other, the pharmaceutical composition comprising valsartan and rosuvastatin calcium according to the present invention can mitigate a side effect(s) of the existing single formulation; improve compliance of patients having hypertension, hyperlipidemia and complex thereof; improve the treatment and prevention effect; and in particular, provide a synergistic effect for treating hypertension.

Furthermore, according to the preparation method of the present invention, the complex formulation is constituted by granulating valsartan and post-mixing rosuvastatin calcium, by which the problem of delaying dissolution due to simple mixing of valsartan and rosuvastatin calcium is overcome and the treatment effect increases.

### [BRIEF EXPLANATION OF THE DRAWINGS]

Figure 1 is a graph comparing and showing the dissolution rates over time for rosuvastatin calcium of the complex formulations of Examples (Ex.) 1 to 4 according to the present invention and the formulation of Comparative Example (CEx.) 1, resulting from the dissolution test thereof.
Figure 2 is a graph comparing and showing the dissolution rates over time for valsartan of the complex formulations of Examples (Ex.) 1 to 4 according to the present invention and the formulation of Comparative Example (CEx.) 1, resulting from the dissolution test thereof.
Figure 3 is a graph comparing and showing the dissolution rates over time for rosuvastatin calcium of the complex formulations of Examples (Ex.) 5 to 8 according to the present invention and the formulation of Comparative Example (CEx.) 1, resulting from the dissolution test thereof.
Figure 4 is a graph comparing and showing the dissolution rates over time for valsartan of the complex formulations of Examples (Ex.) 5 to 8 according to the present invention and the formulation of Comparative Example (CEx.) 1, resulting from the dissolution test thereof.
Figure 5 is a graph showing that the combined administration of valsartan and rosuvastatin according to the present invention exhibits a synergistic effect in lowering sitDBP as compared with single administration.
Figure 6 is a graph showing that the combined administration of valsartan and rosuvastatin according to the present invention exhibits an additional effect in lowering LDL-C as compared with single administration.

### [BEST MODE TO CARRY OUT THE INVENTION]

The present invention is explained in more detail by the following examples. However, these examples seek to illustrate the present invention only, and the scope of the present invention is not limited thereto.

### Examples 1 to 4: Preparation of complex tablet of valsartan and rosuvastatin calcium

**[Table 1]**

| Ingredients | Ex. 1 | Ex. 2 | Ex. 3 | Ex. 4 |
|---|---|---|---|---|
| Valsartan | 80.0 mg | 80.0 mg | 160.0 mg | 160.0 mg |
| Rosuvastatin calcium | 10.4 mg | 20.8 mg | 10.4 mg | 20.8 mg |
| Microcrystalline cellulose | 22.5 mg | 24.0 mg | 45.0 mg | 45.0 mg |
| Low-substituted hydroxypropyl cellulose | 25.0 mg | 26.0 mg | 50.0 mg | 50.0 mg |
| Crospovidone | 15.0 mg | 15.0 mg | 30.0 mg | 30.0 mg |
| Hydroxypropyl cellulose | 6.0 mg | 6.0 mg | 12.0 mg | 12.0 mg |
| Anhydrous calcium hydrogen phosphate | 13.5 mg | 14.5 mg | 27.0 mg | 27.0 mg |
| Hydrous silicon dioxide | 5.0 mg | 5.2 mg | 10.0 mg | 10.0 mg |
| Magnesium stearate | 2.5 mg | 2.5 mg | 5.0 mg | 5.0 mg |
| Total | 179.9 mg | 194.0 mg | 349.4 mg | 359.8 mg |

The above valsartan compositions were wet-granulated by using distilled water, dried and milled to 30 mesh. The granules were mixed with rosuvastatin calcium and compressed into complex tablets which were then film coated. Opadry coating agent (Colorcon) was used as the film coating material, and the film coating was carried out in the same manner in all the following examples and comparative example.

The mixture was combined for a time of 5 minutes or more so that the disintegrating agent which was added in the process of wet-granulation was sufficiently wetted. Such a sufficient combination time increases porosity of the granules and decreases the disintegration time so that the gelling phenomenon can be prevented. In the following examples, all processes of wet-granulation were carried out in the same manner as above.

### Examples 5 to 8: Preparation of complex bilayer tablet of valsartan and rosuvastatin calcium

**[Table 2]**

| Ingredients | Ex. 5 | Ex. 6 | Ex. 7 | Ex. 8 |
|---|---|---|---|---|
| **Valsartan granule** | | | | |
| Valsartan | 80.0 mg | 80.0 mg | 160.0 mg | 160.0 mg |
| Microcrystalline cellulose | 22.5 mg | 24.0 mg | 45.0 mg | 45.0 mg |
| Low-substituted hydroxypropyl cellulose | 25.0 mg | 26.0 mg | 50.0 mg | 50.0 mg |
| Crospovidone | 15.0 mg | 15.0 mg | 30.0 mg | 30.0 mg |
| Hydroxypropyl cellulose | 6.0 mg | 6.0 mg | 12.0 mg | 12.0 mg |
| Hydrous silicon dioxide | 5.0 mg | 5.2 mg | 10.0 mg | 10.0 mg |
| Magnesium stearate | 2.5 mg | 2.5 mg | 5.0 mg | 5.0 mg |
| Sub-total | 156.0 mg | 158.7 mg | 312.0 mg | 312.0 mg |

| **Rosuvastatin granule** | | | | |
|---|---|---|---|---|
| Rosuvastatin calcium | 10.4 mg | 20.8 mg | 10.4 mg | 20.8 mg |
| Microcrystalline cellulose | 60.0 mg | 60.0 mg | 120.0 mg | 120.0 mg |
| Crospovidone | 8.0 mg | 8.0 mg | 16.0 mg | 16.0 mg |
| Anhydrous calcium hydrogen phosphate | 13.5 mg | 14.5 mg | 27.0 mg | 27.0 mg |
| Magnesium stearate | 1.5 mg | 1.5 mg | 3.0 mg | 3.0 mg |
| Sub-total | 93.4 mg | 104.8 mg | 176.4 mg | 186.8 mg |
| Total | 249.4 mg | 263.5 mg | 488.4 mg | 498.8 mg |

The above valsartan granule compositions were wet-granulated by using distilled water, dried and milled to 30 mesh. In the same manner, the rosuvastatin granule compositions were wet-granulated by using distilled water, dried and milled to 30 mesh.

The valsartan granules and rosuvastatin calcium granules were compressed into bilayer solid administration forms and the bilayer solid tablets as formed were then film coated. Opadry coating agent (Colorcon) was used as the film coating material.

### Comparative Example 1: Preparation of simple mixture tablet of valsartan and rosuvastatin calcium

**[Table 3]**

| Ingredients | CEx. 1 |
|---|---|
| Valsartan | 160.0 mg |
| Rosuvastatin calcium | 20.8 mg |
| Microcrystalline cellulose | 45.0 mg |
| Low-substituted hydroxypropyl cellulose | 50.0 mg |
| Crospovidone | 30.0 mg |
| Hydroxypropyl cellulose | 12.0 mg |
| Anhydrous calcium hydrogen phosphate | 27.0 mg |
| Hydrous silicon dioxide | 10.0 mg |
| Magnesium stearate | 5.0 mg |
| Total | 359.8 mg |

The same amounts of valsartan and rosuvastatin calcium as those of Example 4 were mixed together with excipients, wet-granulated by using distilled water, dried and milled to 30 mesh. The granules were compressed into tablets which were then film coated. Opadry coating agent (Colorcon) was used as the film coating material.

### Test Example 1: Dissolution test of valsartan and rosuvastatin calcium for the formulations of Examples 1 to 4

For the complex tablets prepared in Examples 1 to 4 and the tablet prepared in Comparative Example 1, dissolution tests were performed under the following conditions.

### <Conditions for dissolution>

Eluate: The first solution of Disintegration Test in General Test Methods of the Korean Pharmacopoeia (pH 1.2)
Eluate volume: 900 mL
Instrument: USP Paddle Method, 50 rpm
Temperature: 37°C ± 0.5°C
<Conditions for analysis>
Column: Capcellpack C18 type ACR, 4.5 mm LD.×150 mm, 5 µm (or an equivalent thereof)
Mobile phase: Acetonitrile/Distilled water/Acetic acid = 500/500/1 (v/v/v)
Column temperature: 25°C
Flow rate: About 1.0 mL/min
Injection volume: 20 µl
Detector: UV spectrophotometer (Measurement wavelength: 242 nm)

### <Results>

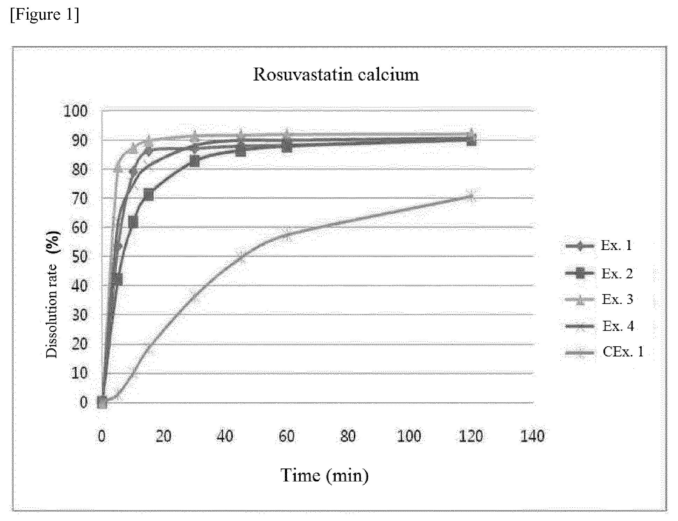

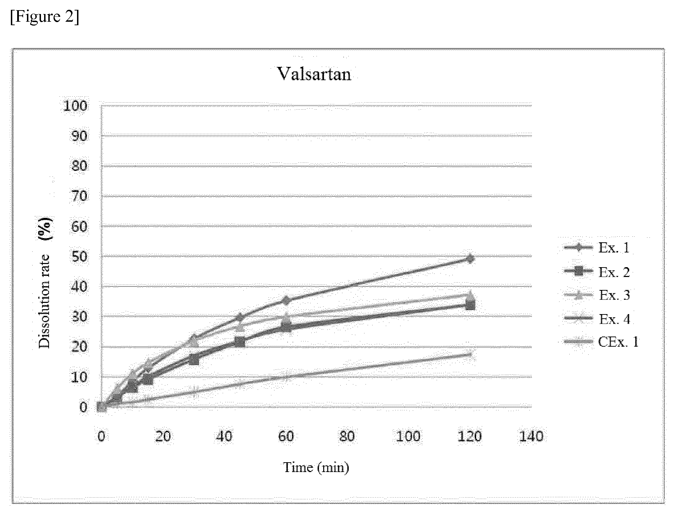

Figures 1 and 2 are graphs comparing and showing the dissolution rates over time for rosuvastatin calcium and valsartan of the complex formulations of Examples (Ex.) 1 to 4 according to the present invention and the formulation of Comparative Example (CEx.) 1, resulting from the dissolution test thereof.

As confirmed by Figures 1 and 2, the dissolution rates for rosuvastatin calcium and valsartan of the complex formulations of Examples 1 to 4, wherein the formulations were prepared by wet-granulating valsartan and post-mixing rosuvastatin calcium thereto, showed an outstandingly high level, as compared with that of the tablet of Comparative Example 1 which was prepared by simply mixing valsartan and rosuvastatin calcium.

### Test Example 2: Dissolution test of valsartan and rosuvastatin calcium for the formulations of Examples 5 to 8

For the complex bilayer tablets prepared in Examples 5 to 8 and the tablet prepared in Comparative Example 1, dissolution tests were performed under the same conditions as those of Test Example 1.

### <Results>

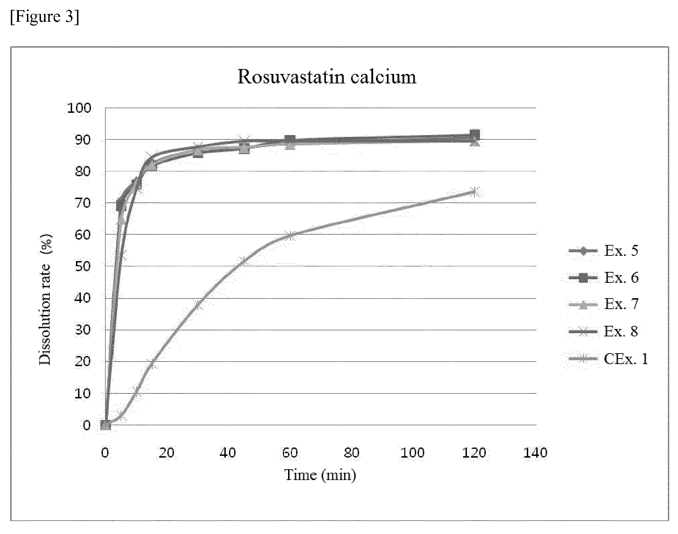

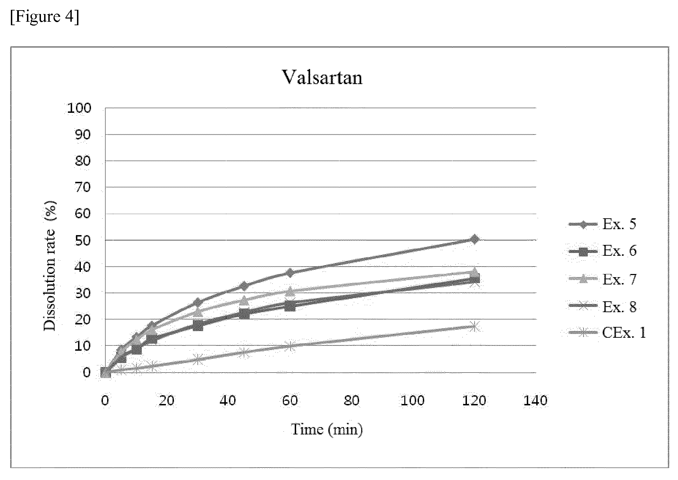

Figures 3 and 4 are graphs comparing and showing the dissolution rates over time for rosuvastatin calcium and valsartan of the complex bilayer tablets of Examples (Ex.) 5 to 8 according to the present invention and the formulation of Comparative Example (CEx.) 1, resulting from the dissolution test thereof.

As confirmed by Figures 3 and 4, the dissolution rates for rosuvastatin calcium and valsartan of the complex bilayer tablets of Examples 5 to 8, wherein the formulations were prepared by wet-granulating valsartan and rosuvastatin calcium separately and compressing the granules into a bilayer tablet, showed an outstandingly high level, as compared with that of the tablet of Comparative Example 1 which was prepared by simply mixing valsartan and rosuvastatin calcium.

### Test Example 3: Clinical effect by the combined administration of valsartan and rosuvastatin

The following tests were performed for patients with hypertension and hyperlipidemia to evaluate the efficacy and safety of the combined administration of 160 mg of valsartan and 20 mg of rosuvastatin in comparison with single administration of each of them.

A screening inspection was conducted for the subjects, who were 20- to 80-year-old patients suffering from hypertension and hyperlipidemia, to ascertain whether they met the selection/exclusion criteria, and administration of the drugs for treating hypertension and hyperlipidemia, which the subjects had taken, was stopped. All the patients who passed the screening inspection were subjected to Therapeutic Lifestyle Change (TLC) for 4 to 6 weeks, and their blood pressure and fasting serum lipid level were measured finally in the measurement 2 (0 week) visit. If the measured values were recognized as suitable to the following criteria, the patient was randomly assigned to one group of the four (4) treatment groups.
1) Blood pressure: 90 mmHg or higher of sitDBP (diastolic blood pressure) (provided that if the patient falls on group D, "high risk," in the following selection criteria on LDL-C value, his/her sitDBP should be 80 mmHg or higher.)
2) Serum lipid: LDL-C (low-density lipoprotein cholesterol)

**[Table 4]**

| Risk group classification | LDL-C value | |
|---|---|---|
| | Based on screening | Based on measurement 2 (Day 0) |
| A. Lower risk: | 160 mg/dL or higher | 160 mg/dL or higher |
| 0~1 cardiovascular risk factor * | | |
| B. Moderate risk: | 130 mg/dL or higher | 160 mg/dL or higher |
| 2 or more cardiovascular risk factors, less than 10% of 10-year risk | | |
| C. Moderately high risk: | 130 mg/dL or higher | 130 mg/dL or higher |
| 2 or more cardiovascular risk factors, from 10% to 20% of 10-year risk | | |
| D. High risk: | 100 mg/dL or higher | 100 mg/dL or higher |
| Risk factor for coronary artery disease or equivalent thereof **, greater than 20% of 10-year risk | | |

| | | |
|---|---|---|
| * Cardiovascular risk factors - Smoking - Hypertension: 140 mmHg or higher of SBP, or 90 mmHg or higher of DBP, or taking antihypertensive drugs - Low HDL-C: Lower than 40 mg/dL (If HDL-C is 60 mg/dL or higher, it is regarded as a protection factor and the total number of risk factors decreases by 1.) - Age: 45 years or older for men, 55 years or older for women - Family history of early-onset of coronary artery disease: In case coronary artery disease occurred in a man younger than 55 years or a woman younger than 65 years among parents, brother(s) and sister(s) ** Coronary artery disease includes myocardial infarction, unstable angina pectoris, coronary artery surgery (angioplasty or bypass) or condition of myocardial ischemia having clinical significance. | | |

Risk factor for coronary artery disease or equivalent thereof includes the cases of atherosclerotic disease (peripheral vascular disease, abdomical aortic aneurysm, carotid disease [transient cerebral ischemia, or stroke due to carotid, or carotid artery stenosis of 50% or more]), diabetes, 20% or greater of 10-year risk for coronary artery disease and 2 or more risk factors.

According to random assignment, each subject was assigned with the same probability to one of a total of four groups consisting of one combined administration group (group 1), two single administration groups (groups 2 and 3) and one placebo group (group 4) as shown in the following table. During the total test period of 12 to 14 weeks, screening visit was done (from 0 week to 6 weeks or 4 weeks), TLC period was conducted, and 0-week, 4-week and 8-week visits were done during the administration period of 8 weeks after the random assignment. The visit window was ± 4 days at each visit, and the timing of visit was determined with the standard of 0 week. The subjects took the drug in a double blind during the measurement and administration periods. At that time, the commercially available Diovan^{®} film coating tablet (160 mg) was used as valsartan and the commercially available Crestor^{®} tablet (20 mg) was used as rosuvastatin. In order to maintain the double blind, placebo drugs corresponding to each of the single preparations were produced so that their appearance could not be distinguished from the two test drugs.

**[Table 5]**

| Measurement 1 (-42 ~ -28 days) | Therapeutic Lifestyle Change (TLC) | Measurement 2 (0 day) | | Measurement 3 (28±4 days) | Measurement 4 (56±4 days) |
|---|---|---|---|---|---|
| -6 ~ -4 weeks | (conducted for 4-6 weeks) | 0 week | | 4 weeks | 8 weeks |
| Screening period | | Administration period in double blind | | | |
| Wash-out | | Group 1 | valsartan (V) 160 mg + rosuvastatin (R) 20 mg | | |
| | | Group 2 | valsartan (V)160 mg + rosuvastatin placebo (R) 20 mg | | |
| | | Group 3 | valsartan placebo (V) 160 mg + rosuvastatin (R) 20 mg | | |
| | | Group 4 | valsartan placebo (V) 160 mg + rosuvastatin placebo (R) 20 mg | | |

At each measuring point, the blood pressure of the subject was measured according to the blood pressure monitoring guidelines provided by the Korean Society of Hypertension to calculate the change of DBP from the baseline after 8 weeks (values after 8 weeks - baseline), and LDL-C was measured and analyzed in the Central Laboratory by a standardized method to calculate the change of LDL-C from the baseline after 8 weeks (values after 8 weeks - baseline). The effects of the single administration and combined administration were compared. The results are shown in Table 6.

**[Table 6]**

| Combined formulation | Single formulation | sitDBP | | LDL-C | |
|---|---|---|---|---|---|
| | | Diff. | Two-sided 95% CI | Diff. | Two-sided 95% CI |
| V160+R20 | R20 | -7.56 mmHg | -10.76, -4.37 | | |
| V160+R20 | V160 | | | -47.68% | -54.1, -41.25 |

The above Table 6 provides the Least Square Mean, which is the corrected average estimated through covariance analysis to the amount of change of sitDBP and rate of change of LDL-C for each test, for each group, and by obtaining two-sided 95% confidence interval (CI) for the average blood pressure difference and rate of change of LDL-C between the combined administration group, and the single administration group and the placebo group, it is proven that a significant difference is recognized when the upper limit of the confidence interval is less than 0. Concretely, the average amount of change of sitDBP is -11.21 mmHg for the combined administration group and -3.64 mmHg for the rosuvastatin group, and the difference between the groups is -7.56 mmHg, and the 95% confidence interval for the difference is (-10.76, -4.37) where the upper limit of the confidence interval is less than 0, from which it is confirmed that the combined administration group shows a superior effect in lowering sitDBP as compared with the single administration group of rosuvastatin. The average rate of change of LDL-C is -52.19% for the combined administration group and - 4.52% for the valsartan group, and the difference between the groups is -47.68%, and the 95% confidence interval for the difference is (-54.1, -41.25) where the upper limit of the confidence interval is less than 0, from which it is confirmed that the combined administration group shows a superior effect in lowering LDL-C as compared with the single administration group of valsartan.

That is, it is statistically proven that after 8 weeks the combined administration group of rosuvastatin and valsartan shows superiority in the amount of change of sitDBP from the baseline, over the single administration group of rosuvastatin. It is also statistically proven that this combined administration group shows superiority in the rate of change of LDL-C over the single administration group of valsartan.

Additionally, the following Table 7 shows the difference in the corrected amount change of sitDBP after 8 weeks from the baseline between the combined administration group and single administration group of valsartan.

**[Table 7]**

| Combined formulation (V160+R20) | Single formulation (V160) | sitDBP | |
|---|---|---|---|
| | | Diff. | Two-sided 95% CI |
| -11.15 mmHg | -7.26 mmHg | -3.89 mmHg | -7.1,-0.68 |

As can be seen from the results of Table 7, between the two groups the difference in the amount change of sitDBP is -3.89 mmHg which is statistically significant, and thus it is confirmed that the combined administration of valsartan and rosuvastatin has the additional efficacy of lowering blood pressure, as compared with the single administration of valsartan.

The following Table 8 shows the corrected average rate of change of LDL-C after 8 weeks from the baseline for the combined administration group and single administration group of rosuvastatin.

**[Table 8]**

| Combined formulation (V160+R20) | Single formulation (R20) | LDL-C | |
|---|---|---|---|
| | | Diff. | Two-sided 95% CI |
| -51.82% | -47.27% | -4.55% | -10.92, 1.81 |

As can be seen from the results of Table 8, the difference between the combined administration group and single administration group of rosuvastatin is -4.55% and the additional efficacy of reducing LDL-C is shown in the combined administration group, but no statistically significant difference between the two groups is confirmed.

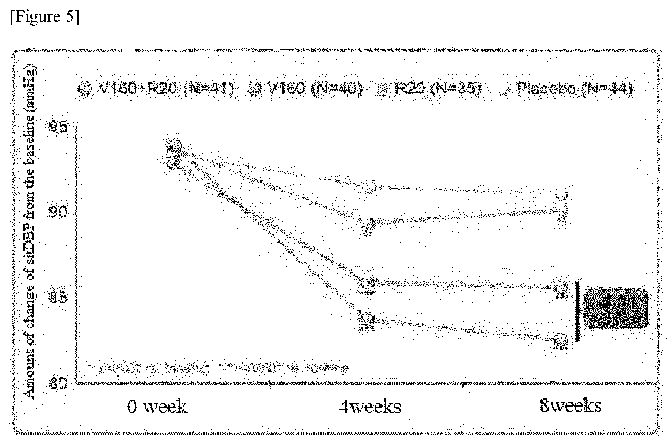

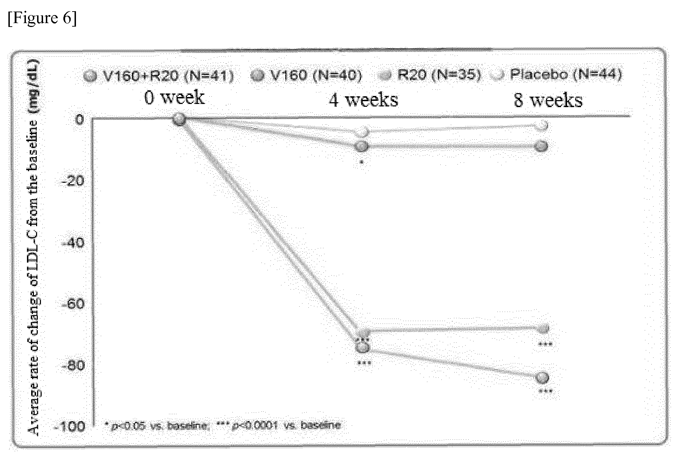

Figure 5 is a graph showing that the combined administration of valsartan and rosuvastatin according to the present invention exhibits a synergistic effect in lowering sitDBP as compared with single administration, and Figure 6 is a graph showing that the combined administration of valsartan and rosuvastatin according to the present invention exhibits an additional effect in lowering LDL-C as compared with single administration.

## Claims

1. A pharmaceutical composition for preventing or treating a cardiovascular disease and a hyperlipidemic disease, comprising valsartan and rosuvastatin calcium as active ingredients.

2. The pharmaceutical composition according to claim 1, wherein the valsartan and rosuvastatin calcium are present in a state of being separated.

3. The pharmaceutical composition according to claim 2, wherein the rosuvastatin calcium is in a state of being mixed to valsartan granule.

4. The pharmaceutical composition according to claim 3, further comprising a pharmaceutically acceptable additive in addition to the valsartan granule and rosuvastatin calcium.

5. The pharmaceutical composition according to claim 2, which is in a form of bilayer tablet where each of the valsartan and rosuvastatin calcium is present in each of the separate layers.

6. The pharmaceutical composition according to claim 1, comprising the rosuvastatin and valsartan in a weight ratio of from 1:2 to 1:32.

7. The pharmaceutical composition according to claim 6, comprising the rosuvastatin and valsartan in a weight ratio of 1:4, 1:8, 1:16 or 1:32.

8. The pharmaceutical composition according to claim 7, wherein the pharmaceutical composition is a tablet and comprises, per tablet, 5 mg of rosuvastatin + 80 mg of valsartan, 5 mg of rosuvastatin + 160 mg of valsartan, 10 mg of rosuvastatin + 80 mg of valsartan, 10 mg of rosuvastatin + 160 mg of valsartan, 20 mg of rosuvastatin + 80 mg of valsartan, or 20 mg of rosuvastatin + 160 mg of valsartan.

9. The pharmaceutical composition according to claim 1, for preventing or treating one or more diseases selected from angina pectoris, hypertension, hyperlipidemia, arteriospasm, cardiac arrhythmia, cardiac hypertrophy, cerebral infarction, congestive heart failure and myocardial infarction.

10. A method for preparing a pharmaceutical composition for preventing or treating a cardiovascular disease and a hyperlipidemic disease, comprising the steps of:
(a) granulating a mixture of valsartan and a pharmaceutically acceptable additive; and
(b) mixing rosuvastatin calcium and a pharmaceutically acceptable additive to the valsartan granule of step (a).

11. The preparation method according to claim 10, wherein a mixture of 1 part by weight of the valsartan and 1 to 3 parts by weight of the pharmaceutically acceptable additive is granulated in said step (a).

12. The preparation method according to claim 10, wherein 1 part by weight of the rosuvastatin calcium and 1 to 10 parts by weight of the pharmaceutically acceptable additive are mixed in said step (b).

13. A pharmaceutical composition for synergistically treating hypertension, comprising valsartan and rosuvastatin calcium as active ingredients.

14. The composition according to claim 13, wherein the composition is a tablet and comprises, per tablet, 160 mg of valsartan and 20 mg of rosuvastatin.

15. Use of a pharmaceutical composition comprising valsartan and rosuvastatin calcium as active ingredients for preventing or treating a cardiovascular disease and a hyperlipidemic disease.

16. A method of preventing or treating a cardiovascular disease and a hyperlipidemic disease, comprising a step of administering a pharmaceutical composition comprising valsartan and rosuvastatin calcium as active ingredients to a subject in need.
